# EUROPÄISCHE PATENTSCHRIFT

(11) **EP 0 755 949 B1**
(45) Veröffentlichungstag und Bekanntmachung des Hinweises auf die Patenterteilung: **12.04.2000**
(21) Anmeldenummer: 96111408.9
(22) Anmeldetag: 16.07.1996
(51) Int. Cl.: C08F 2/50, C09D 4/00, C08F 299/02, C07C 323/22, C07C 49/82, C07C 225/16

(54) **Strahlungshärtbare Massen mit kovalent gebundenen Photoinitiatoren**
Radiation curable masses containing covalently bonded photoinitiators
Masses durcissables par irradiations contenant des photo-initiateurs liés de manière covalente

(30) Priorität: 22.07.1995 DE 19526856
(43) Veröffentlichungstag der Anmeldung: 29.01.1997
(73) Patentinhaber: BASF AKTIENGESELLSCHAFT, 67056 Ludwigshafen (DE)
(72) Erfinder: Schröder, Jochen, Dr., 09701-970 Sao Bernardo do Campo (BR); Reich, Wolfgang, Dr., 67133 Maxdorf (DE); Beck, Erich, Dr., 68528 Ladenburg (DE); Fischer, Martin, Dr., 67071 Ludwigshafen (DE); Weiss, Wolfram, Dr., 67112 Mutterstadt (DE)

(56) Entgegenhaltungen:
- EP-A- 0 000 342
- EP-A- 0 005 530
- EP-A- 0 143 201
- WO-A-93/21240
- DE-A- 2 001 288
- US-A- 4 180 599
- DATABASE BEILSTEIN XP002016343 & PHARMAZIE, Bd. 24, 1969, Seiten 378-382, KAZMIROWSKI: "Synthese antiproteolytisch wirksamer Derivate des Benzylamins und des Benzamidins"

## Beschreibung

Die Erfindung betrifft ein Verfahren zur Herstellung von strahlungshärtbaren Acrylatzusammensetzungen.

Strahlungshärtbare Acrylate sind generell bekannt. Den strahlungshärtbaren Acrylaten wird üblicherweise ein Photoinitiator zugesetzt, um so eine Härtung durch Bestrahlung mit UV-Licht zu bewirken. Nach der Bestrahlung können Reste der Photoinitiatoren als flüchtige oder extrahierbare Substanzen in der gehärteten Masse verbleiben, was generell unerwünscht ist.

Copolymerisierbare Photoinitiatoren bzw. coreaktive Photoinitiatoren sind z.B. aus EP-A-281 941 bekannt. Es handelt sich dabei vorwiegend um Acetophenonderivate, welche als sog. α-Spalter bei der Belichtung in 2 Moleküle zerfallen, von denen nur eines über reaktive Gruppen an das Polymer fixiert ist bzw. wird.

Aus EP-A-280 222 ist bekannt, daß die Reaktivität von strahlungshärtbaren Massen auf Basis von Acrylaten durch Zusatz von primären oder sekundären Aminen erhöht werden kann. Die primären oder sekundären Amine addieren sich dabei in Form einer Michael-analogen Addition an die Acryldoppelbindungen.

Aus DE-A-2001288 und Pharmazie 1969 (24), Seiten 378 - 382 sind Benzophenonderivate bekannt, die an Alkylgruppen gebundene Hydroxyl- oder Aminogruppen enthalten. EP-A-342 und EP-A-5530 beschreiben ebenfalls Benzophenonderivate, welche Hydroxyl-, Amino- oder Mercaptogruppen enthalten.

Copolymerisierbare Benzophenonderivate sind in US-A-4180599 sowie EP-A-143201 beschrieben. Die Michaeladdition von Aminogruppen an olefinisch ungesättigte Gruppen ist aus WO-A-9321240 bekannt.

Aufgabe der vorliegenden Erfindung waren strahlungshärtbare Acrylate und Massen mit hoher Reaktivität bei der Strahlungshärtung und geringen Mengen an flüchtigen bzw. extrahierbaren Anteilen.

Demgemäß wurde ein Verfahren zur Herstellung von strahlungshärtbaren Acrylatzusammensetzungen, dadurch gekennzeichnet, daß Verbindungen mit mindestens 2 Acrylgruppen (Verbindungen A) nicht radikalisch copolymerisierbare Benzophenonderivate zugesetzt werden, die mindestens eine primäre oder sekundäre Aminogruppe enthalten, gefunden. Gefunden wurden auch strahlungshärtbare Massen, die durch ein solches Verfahren erhältlich sind. Ebenso wurden Benzophenonderivate gefunden, welche für das vorstehende Verfahren geeignet sind.

Die nachfolgenden Ausführungen betreffen bevorzugte Ausgestaltungen der Erfindung.

Wesentlicher Bestandteil der strahlungshärtbaren Massen sind Verbindungen A mit mindestens zwei Acrylgruppen. An die Acrylgruppen können sich die primäre oder sekundäre Aminogruppe der Benzophenonderivate in Form einer Michael-analogen Addition gemäß 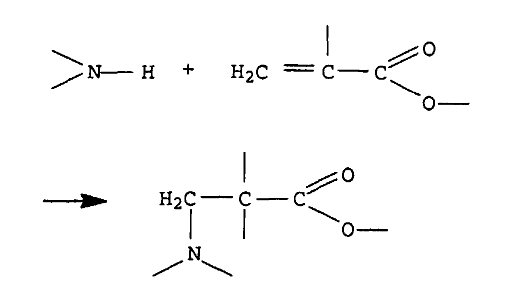 anlagern.

Aus primären Aminogruppen werden dabei sekundäre Aminogruppen, aus sekundären tertiäre Aminogruppen.

Unter den Verbindungen A werden neben Derivaten der Acrylsäure auch solche der Methacrylsäure verstanden. Die Derivate der Acrylsäure sind jedoch bevorzugt.

Die Verbindungen A enthalten im Mittel bevorzugt 2 bis 20, besonders bevorzugt 2 bis 10 und ganz besonders bevorzugt 2 bis 6 Acrylgruppen im Molekül.

Das zahlenmittlere Molekulargewicht Mₙ der Verbindungen A liegt bevorzugt unter 15000, besondere bevorzugt unter 5000, und ganz besonders bevorzugt unter 3000 g/mol (bestimmt durch Gelpermeationschromatographie mit Polystyrol als Standard und Tetrahydrofuran als Elutionsmittel).

Als Verbindungen A genannt seien z.B. Acrylsäureester von mehrfunktionellen Alkoholen, insbesondere solchen, die neben den Hydroxylgruppen keine weiteren funktionellen Gruppen oder allenfalls Ethergruppen enthalten. Beispiele solcher Alkohole sind z.B. bifunktionelle Alkohole, wie Ethylenglykol, Propylenglykol, und deren höher kondensierte Vertreter, z.B. wie Diethylenglykol, Triethylenglykol, Dipropylenglykol, Tripropylenglykol etc., Butandiol, Pentandiol, Hexandiol, Neopentylglykol, alkoxylierte phenolische Verbindungen, wie ethoxylierte bzw. propoxylierte Bisphenole, Cyclohexandimethanol, trifunktionelle und höherfunktionelle Alkohole, wie Glycerin, Trimethylolpropan, Butantriol, Trimethylolethan, Pentaerythrit, Ditrimethylolpropan, Dipentaerythrit, Sorbit, Mannit und die entsprechenden alkoxylierten, insbesondere ethoxy- und propoxylierte Alkohole.

Die Alkoxylierungsprodukte sind in bekannter Weise durch Umsetzung der vorstehenden Alkohole mit Alkylenoxiden, insbesondere Ethylen- oder Propylenoxid, erhältlich. Vorzugsweise beträgt der Alkoxylierungsgrad je Hydroxylgruppe 0 bis 10, d.h. 1 mol Hydroxylgruppe kann vorzugsweise mit bis zu 10 mol Alkylenoxiden alkoxyliert sein.

Als Acrylatverbindungen seien weiterhin Polyesteracrylate genannt, wobei es sich um die Acrylsäureester von Polyesterolen handelt.

Als Polyesterole kommen z.B. solche in Betracht, wie sie durch Veresterung von Polycarbonsäuren, vorzugsweise Dicarbonsäuren, mit Polyolen, vorzugsweise Diolen, hergestellt werden können. Die Ausgangsstoffe für solche hydroxylgruppenhaltige Polyester sind dem Fachmann bekannt. Bevorzugt können als Dicarbonsäuren Bernsteinsäure, Glutarsäure, Adipinsäure, Sebacinsäure, o-Phthalsäure, deren Isomere und Hydrierungsprodukte sowie veresterbare Derivate, wie Anhydride oder Dialkylester der genannten Säuren eingesetzt werden. Als Polyole kommen die oben genannten Alkohole, vorzugsweise Ethylenglykol, Propylenglykol-1,2 und -1,3, Butandiol-1,4, Hexandiol-1,6, Neopentylglykol, Cyclohexandimethanol sowie Polyglykole vom Typ des Ethylenglykols und Propylenglykols in Betracht.

Polyesteracrylate können in mehreren Stufen oder auch einstufig, wie z.B. in EP 279 303 beschrieben, aus Acrylsäure, Polycarbonsäure, Polyol hergestellt werden.

Weiterhin kann es sich bei Verbindungen A z.B. um Epoxid- oder Urethanacrylate handeln.

Epoxid(meth)acrylate sind z.B. solche wie sie durch Umsetzung von epoxidierten Olefinen oder Poly- bzw. Diglycidylethern, wie Bisphenol-A-diglycidylether, mit (Meth)acrylsäure erhältlich sind.

Die Umsetzung ist dem Fachmann bekannt und z.B. in R. Holmann, U.V. and E.B. Curing Formulation for Printing Inks and Paints, London 1984, beschrieben.

Bei Urethanacrylaten handelt es sich insbesondere um Umsetzungsprodukte von Hydroxyalkylacrylaten mit Poly- bzw. Diisocyanaten (s. ebenfalls R. Holmann, U.V. and E.B. Curing Formulation for Printing Inks and Paints, London 1984).

Es können natürlich auch Mischungen verschiedener Verbindungen A eingesetzt werden.

Den Verbindungen A werden erfindungsgemäß die Benzophenonderivate zugesetzt. Hierbei erfolgt eine Michael-Addition, ohne daß die Temperatur erhöht werden muß. Durch höhere Temperaturen kann die Michael-Addition beschleunigt werden. Die Temperatur wird im allgemeinen auf 0 bis 200°C, bevorzugt 15 bis 80°C gehalten.

Bei den Benzophenonderivaten handelt es sich um Photoinitiatoren für die radikalische Polymerisation.

Die Benzophenonderivate enthalten eine primäre oder sekundäre Aminogruppe.

Insbesondere handelt es sich um Benzophenonderivate der Formel 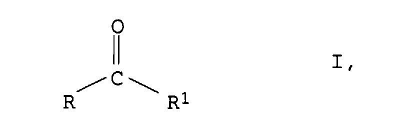 in der R für einen Phenylrest oder für R¹ steht und R¹ für den Rest 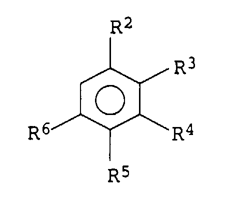 steht, wobei die Reste R² bis R₆ unabhängig voneinander für H, eine C₁-C₄-Alkyl- oder C₁-C₄-Alkoxy-gruppe stehen und mindestens einen, maximal drei der Reste R³ bis R⁶ für eine Gruppe X steht und X eine organische Gruppe mit 1 bis 30 C-Atomen und mit einer primären oder sekundären Aminogruppe bedeutet.

Bevorzugt handelt es sich bei den Resten R² bis R⁶, soweit sie nicht für eine Gruppe X stehen, um ein H-Atom.

Insbesondere steht nur einer der Reste R² bis R⁶ für eine Gruppe X, besonders bevorzugt steht R⁴ für eine Gruppe X.

Bei X handelt es sich z.B. um eine aromatische oder insbesondere um eine aliphatische Gruppe mit 1 bis 20, besonders bevorzugt 1 bis 10 C-Atomen mit einer primären oder sekundären Aminogruppe.

R steht bevorzugt für einen Phenylrest.

Als Beispiele für die erfindungsgemäßen Benzophenonderivate seien folgende Verbindungen aufgeführt:

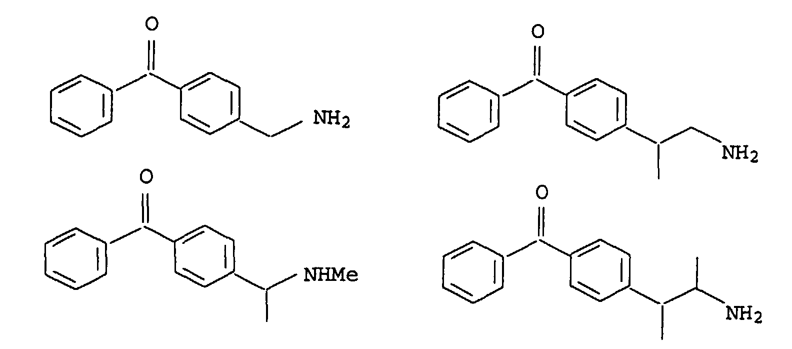

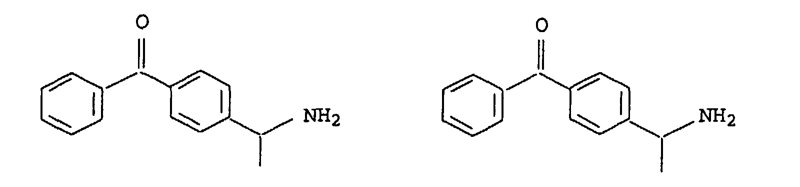

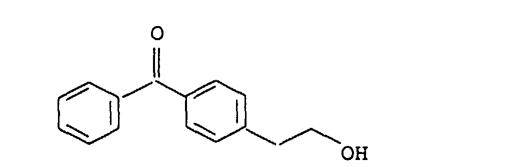

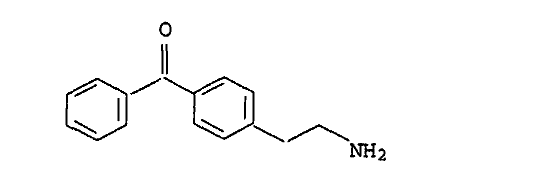

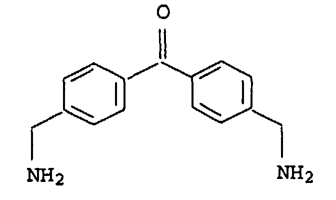

Zur Herstellung der erfindungsgemäßen Benzophenonderivate sind verschiedene Verfahren I bis III möglich.

### Verfahren I

Dieses Verfahren ist dadurch gekennzeichnet, daß
- von einer Verbindung der Formel I, worin jedoch mindestens einer, maximal drei der Reste R³ bis R⁶ ein C₁-C₂₀-Alkylrest statt einer Gruppe X sind, ausgegangen wird,
- der Alkylrest mit elementarem Chlor chloriert wird und anschließend
- mit Natriumdiformylamid umgesetzt und hydrolysiert wird.

Die Chlorierung mit elementarem Chlor erfolgt vorzugsweise bei Temperaturen von 0 bis 100°C, so daß nur der Alkylrest chloriert wird.

Das erhaltene Produkt wird dann mit einem vorzugsweise bei Temperaturen von 0 bis 100°C mit einem Natriumdiformylamid umgesetzt, wobei eine Substitution des Chlors durch eine Formylaminogruppe gemäß 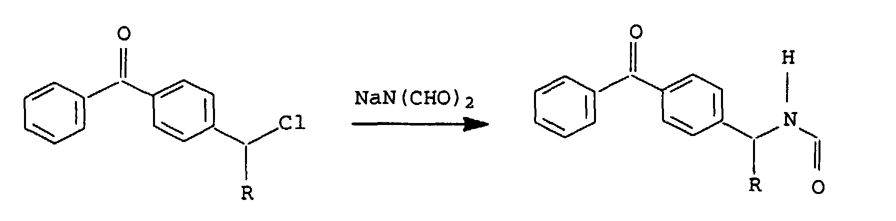 eintritt.

Durch anschließende Hydrolyse mit Wasser eintritt kurz R-Y erhält man dann das gewünschte Endprodukt gemäß

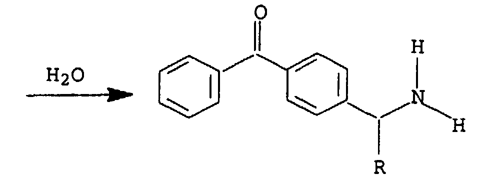

### Verfahren II

Dieses Verfahren ist dadurch gekennzeichnet, daß
- ein Benzotrichloridderivat mit einem Aromaten der Formel 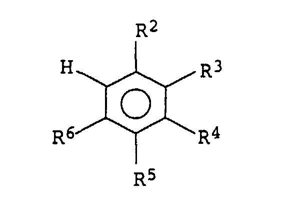 zu einem Benzophenonderivat umgesetzt wird, worin die Variablen die oben angegebene Bedeutung haben, jedoch mindestens einer, maximal drei der Reste R³ bis R₆ für einen organischen Rest mit einer tertiären, mit einer Schutzgruppe maskierten Aminogruppe statt für X stehen und
- die Schutzgruppe durch saure Hydrolyse abgespalten wird.

Die Umsetzung zum Benzophenonderivat wird vorzugsweise bei Temperaturen von -20 bis +20°C und in Gegenwart eines Katalysators, z.B. Aluminiumchlorid oder ein Zeolith durchgeführt. Bei der Schutzgruppe am tertiären Amin handelt es sich bevorzugt um einen Acetylrest.

Die Schutzgruppe kann abschließend in bekannter Weise z.B. durch saure Hydrolyse abgespalten werden, wobei ein primäres oder sekundäres Amin in der Seitenkette erhalten wird.

### Verfahren III

Dieses Verfahren ist dadurch gekennzeichnet, daß ein Aromat mit der Formel 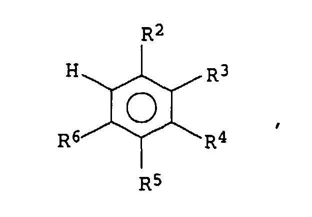 worin die Variablen die oben genannte Bedeutung haben, jedoch mindestens einer maximal drei der Reste R³ bis R⁶ für einen organischen Rest mit einer Carbonylgruppe statt für X stehen, mit einer aliphatischen Nitroverbindung kondensiert wird
- die erhaltene Verbindung mit einem Benzotrichloridderivat zu einem Benzophenonderivat umgesetzt wird und
- die Nitrogruppe anschließend hydriert wird.

Bei diesem Verfahren wird zunächst eine aromatische Carbonylverbindung mit einer Nitroverbindung, vorzugsweise einer aliphatischen Nitroverbindung gemäß 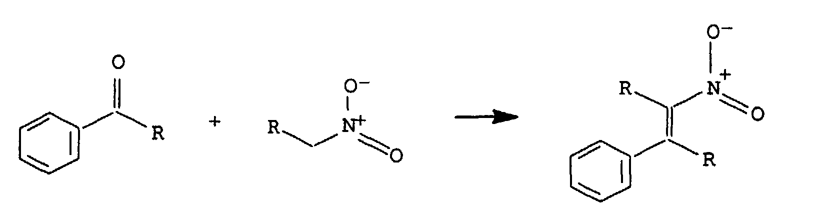 vorzugsweise bei Temperaturen von 50 bis 150°C kondensiert.

Die Doppelbindung wird anschließend mit Wasserstoff hydriert 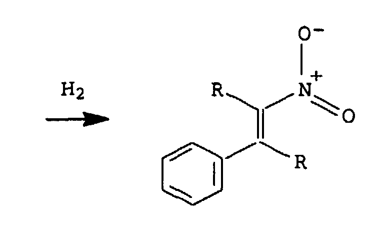 und das Produkt wie unter II mit einem Trichlorbenzolderivat umgesetzt gemäß

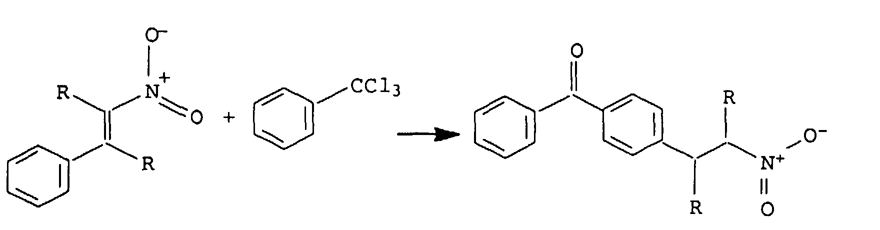

Die Nitrogruppe im erhaltenen Benzophenonderivat wird anschließend mit Wasserstoff zur Aminogruppe hydriert:

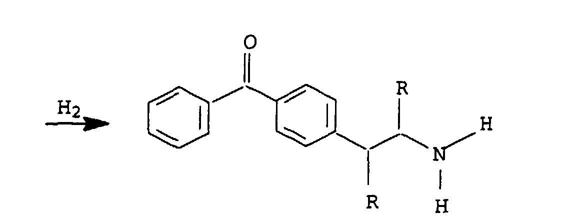

Durch die Zugabe der Benzophenonderivate zu den Verbindungen A sind strahlungshärtbare Acrylatzusammensetzungen mit kovalent (durch Michael-Addition gebundene Photoinitiatoren) erhältlich. Die Menge der Benzophenonderivate sollte dabei so bemessen sein, daß vorzugsweise nicht mehr als 70 mol%, besonders bevorzugt nicht mehr als 50 mol%, ganz besonders bevorzugt nicht mehr als 30 mol% der Acrylgruppen der Verbindungen A als Michael-Addukt mit den Benzophenonderivaten vorliegen, so daß noch genügend Doppelbindungen für die radikalische Polymerisation vorliegen.

Im allgemeinen sollte die Menge jedoch so bemessen sein, daß mindestens 0,1 mol %, vorzugsweise mindestens 0,5 mol % der Acrylgruppen als Michael-Addukt mit den Benzophenonderivaten vorliegen.

Die erfindungsgemäßen strahlungshärtbaren Acrylatzusammensetzungen können natürlich auch mit anderen radikalisch polymerisierbaren Verbindungen im Gemisch als strahlungshärtbare Massen verwendet werden.

Vorzugsweise enthalten die strahlungshärtbaren Massen 1 bis 100 Gew.-%, besonders bevorzugt 20 bis 100 und ganz besonders bevorzugt 50 bis 100 Gew.-% der erfindungsgemäßen strahlungshärtbaren Acrylatzusammensetzungen A, bezogen auf die Gesamtmenge an radikalisch copolymerisierbaren Verbindungen.

Als andere radikalisch copolymerisierbaren Verbindungen kommen z.B. Monoacrylate z.B. C₁-C₁₀-Alkyl(meth)acrylate, Vinylaromaten, z.B. Styrol, Vinylester, z.B. Vinylacetat, Vinylhalogenide, Vinylnitrile, aber auch copolymerisierbare oligomere oder auch polymere Verbindungen in Betracht.

Die anderen radikalisch copolymerisierbaren Verbindungen können bereits Verbindungen A vor der Zugabe der Benzophenonderivate zugesetzt werden.

Bevorzugt werden die anderen radikalisch copolymerisierbaren Verbindungen nach der Zugabe der Benzophenonderivate, also nachdem die erfindungsgemäßen strahlungshärtbaren Acrylatzusammensetzungen erhalten wurden, zugesetzt.

Der Gehalt der Benzophenonderivate in den strahlungshärtbaren Massen beträgt vorzugsweise 0,1 bis 10 Gew.-%, bezogen auf die Gesamtmenge der radikalisch copolymerisierbaren Verbindungen. Besonders bevorzugt ist ein Gehalt von 0,5 bis 5 Gew.-% (wobei die Gewichtsmenge der Benzophenonderivate wie zugesetzt, also in ungebundener Form berechnet wird).

Die erfindungsgemäßen strahlungshärtbaren Massen bzw. Acrylatzusammensetzungen eignen sich z.B. als Überzugsmassen, wie Lacken, Druckfarben oder zur Herstellung von Formkörpern, zur Herstellung von Photoresisten, in der Stereolithographie oder als Gießmasse, z.B. für optische Linsen.

Die Massen bzw. Acrylatzusammensetzungen können weitere Zusatzstoffe, wie sie z.B. für die jeweilige Verwendung üblich sind, enthalten.

Bei der bevorzugten Verwendung als Beschichtungsmasse seien z.B. Verdicker, Verlaufsmittel, Füllstoffe oder Pigmente genannt.

Die Massen bzw. Acrylatzusammensetzungen werden vorzugsweise durch Bestrahlung mit UV-Licht gehärtet und zeigen hierbei eine hohe Reaktivität. Die erhaltenen Beschichtungen bzw. sonstigen Produkte haben gute mechanische Eigenschaften, eine hohe Härte und enthalten kaum flüchtige oder extrahierbare Photoinitiator-bzw. Benzophenonbestandteile.

### Beispiele

### Herstellung von Benzophenonderivaten

### A) 4-Aminomethylbenzophenon (Verfahren I)

i) 4-(Chlormethyl)benzophenon
   196,2 g 4-Methylbenzophenon werden in Chlorbenzol gelöst und bei 80°C in Gegenwart von 1,6 g AIBN Azodiisobutyronitril mit insgesamt 71,0 g Chlor begast. Das Reaktionsgemisch wird eingeengt und der erhaltene Feststoff aus 1 l Ethanol umkristallisiert.
   Ausbeute: 150 g
   UV: λₘₐₓ = 343 nm, ε= 173,9 (in Toluol).
ii) 4-(N-Formylaminomethyl)benzophenon
   150 g Formamid werden zusammen mit 90 ml 30 %ige Natriummethylatlösung 8 Stunden im leichten Vakuum bei 80°C gerührt. Zu der so hergestellten Lösung von Natriumdiformylamid werden 150 g 4-Chlormethylbenzophenon gegeben. Nach 15 Stunden hat sich aus der ursprünglich vorliegenden Suspension eine klare Lösung gebildet, die in 1 l 10 %ige Natriumcarbonatlösung gegeben wird. Es fällt ein Feststoff aus, der abgesaugt und getrocknet wird.
   Ausbeute: 126 g
   UV: λₘₐₓ = 344 nm, ε= 150,5 (in Toluol).
iii) 4-Aminomethylbenzophenon
   70 g 4-(N-Formylaminomethyl)benzophenon werden in einer Mischung aus 40 g konzentrierter Salzsäure und 100 g Ethanol 5 Stunden bei 50°C gerührt. Danach wird der Alkohol abdestilliert und mit Natronlauge pH 10 eingestellt. 4-Aminomethylbenzophenon scheidet sich als Öl ab.
   Ausbeute: 20 g
   UV: λₘₐₓ = 343 nm, ε= 155,8 (in Toluol).

### B) 4-(N-Methylaminomethyl)benzophenon (Verfahren II)

4-(N-Acetyl-N-methylaminomethyl)benzophenon

40 g AlCl₃ werden in 100 ml Dichlorethan gelöst und bei 0 bis 5°C mit 21,5 g Benzotrichlorid versetzt. Dann werden 16,3 g N-Methylbenzylacetamid als Lösung in Dichlorethan zugegeben (Temperatur 0 bis 5°C). Nach beendeter HCl-Entwicklung wird der Ansatz in 500 g Eiswasser gegeben und anschließend bei 70°C hydrolysiert. Aus der organischen Phase werden 30 g Feststoff isoliert, der als solcher in die nächste Stufe eingesetzt wird.

### Alternativ wird wie folgt verfahren:

21,5 g Benzotrichlorid und 16,3 g N-Methylbenzylacetamid werden in 100 ml Dichlorethan gelöst und mit 1 g HZSM-5 (SiO₂/Al₂O₃ = 40) versetzt und bis zum Ende der HCl-Entwicklung am Rückfluß gekocht. Dann werden 500 ml Eiswasser zugegeben und anschließend bei 70°C hydrolysiert. Aus der organischen Phase werden 30 g Feststoff isoliert, der als solcher in die nächste Stufe eingesetzt wird.

### 4-(N-Methylaminomethyl)benzophenon

22 g 4-(N-Acetyl-N-methylaminomethyl)benzophenon werden in 100 ml 1:1-HCl/Wasser-Gemisch (Volumenteile) 7 Stunden am Rückfluß gekocht. Aus dem Reaktionsgemisch werden 16 g 4-(N-Methylaminomethyl)benzophenon isoliert.
HNMR: δ = 1,7 (1H, NH), 2,5 (3H, NCH₃), 3,9 (2H, NCH₂), 7,1 - 7,4 (9H, Aromaten).

### C) 4-(2-Aminoethyl)benzophenon

### 4-(2-N-Acetylaminoethyl)benzophenon

40 g AlCl₃ werden in 100 ml Dichlorethan gelöst und bei 0 bis 5°C mit 21,5 g Benzotrichlorid versetzt. Dann werden 16 g N-Phenylethylacetamid als Lösung in Dichlorethan zugegeben (Temperatur 0 bis 5°C). Nach beendeter HCl-Entwicklung wird der Ansatz in 500 ml Eiswasser gegeben und anschließend bei 70°C hydrolysiert. Aus der organischen Phase werden 30 g Feststoff isoliert, der als solcher in die nächste Stufe eingesetzt wird.

### Alternativ wird wie folgt verfahren:

21,5 g Benzotrichlorid und 16,3 g N-Methylbenzylacetamid werden in 100 ml Dichlorethan gelöst und mit 1 g HZSM-5 (SiO₂/Al₂O₃ = 40) versetzt und bis zum Ende der HCl-Entwicklung am Rückfluß gekocht. Dann werden 500 ml Eiswasser zugegeben und anschließend bei 70°C hydrolysiert. Aus der organischen Phase werden 30 g Feststoff isoliert, der als solcher in die nächste Stufe eingesetzt wird.

### 4-(2-Aminoethyl)benzophenon

22 g 4-(2-N-Acetylaminoethyl)benzophenon werden in 100 ml 1:1-HCl/Wasser-Gemisch (Volumenteile) 7 Stunden am Rückfluß gekocht. Aus dem Reaktionsgemisch werden 16 g 4-(2-Aminoethyl)benzophenon isoliert.
HNMR: δ = 1,3 (2H, NH), 2,9 und 3,1 (je 2H, NCH3), 7,1 - 7,4 (9H, Aromaten).

### Prüfungen

### a) Reaktivität

Die Fotoinitiatoren wurden in Mengenverhältnissen (Gewichtsteile) entsprechend der folgenden Tabelle mit Acrylesterharzen abgemischt. Die weiteren Prüfungen erfolgten nach 24 Stunden Lagerung. Die Viskositäten wurden mit einem Kegel-Platte-Viskosimeter bestimmt. Zur Messung der Härtungsgeschwindigkeit unter UV-Strahlung wurden Lackfilme, aufgezogen mit einem 15 µm Spiralrakel auf Kunstdruckpapier, auf einem Transportband im Abstand von 10 cm unter einem undotierten Quecksilber-Hochdruckstrahler (Firma IST, Typ: CK1) durchgefahren. Bandgeschwindigkeit und Anzahl an Bestrahlungszyklen bestimmen die eingestrahlte Lichtintensität, die zum Erreichen einer klebfreien und kratzfesten Beschichtung benötigt wird.

Je schneller die Bandgeschwindigkeit sein kann und je weniger Bestrahlungszyklen benötigt werden, um klebfreie und kratzfeste Beschichtungen zu bekommen, desto höher ist die Reaktivität.

| Lack | Fotoinitiator | Teile ** | Harz 100 Teile Laromer® | Viskosität | Bandgeschwindigkeit m/min | Bestrahlungszyklen Anzahl |
|---|---|---|---|---|---|---|
| 1 | A | 3,3 | PO 83 F¹ | 110 | 10 | 2 |
| 2 | B | 3 | PO 33 F² | 120 | 10 | 2 |
| 3 | B | 3 | PO 83 F | 130 | 10 | 1 |
| 4 | B | 3,71 | PO 33 F | 95 | 20 | 1 |
| 5 | C | 3 | PO 33 F | 95 | 10 | 6 |
| 6 | C | 3 | PO 83 F | 100 | 10 | 2 |
| 7 | B | 5 | LR 8765³ | 900 | 10 | 1 |
| 8 | B | 5 | LR 8799⁴ | 4600 | 10 | 1 |
| 9 | B | 5 | LR 8945 X⁵ | 650 | 15 | 1 |
| 10* | Benzophenon | 2,4 | Po 83 F | 100 | 15 | 1 |
| Laromer® Warenzeichen der BASF AG | | | | | | |

| | | | | | | |
|---|---|---|---|---|---|---|
| * Vergleichsbeispiel mit zusätzlich 1,6 Teilen Methyldiethanolamin als Coinitiator | | | | | | |
| ** Gewichtsteile bezogen auf Laromer, wobei in Beispielen 1,3,6 und 10 gleich molare Mengen des Photoinitiators und in Beispielen 1 und 10 gleiche molare Mengen Amin vorlagen. | | | | | | |
| ¹ ein aminmodifiziertes Polyetheracrylat | | | | | | |
| ² ein Polyetheracrylat | | | | | | |
| ³ ein aliphatische Epoxidacrylat | | | | | | |
| ⁴ ein Polyesteracrylat | | | | | | |
| ⁵ ein Polyetheracrylat | | | | | | |

### b) Extraktion

### Filmherstellung:

Von den Lacken 7 bis 10 wurden mit einem 200 µm Spiralrakel Filme auf Glasplatten aufgezogen und 5*10 m/min belichtet. Die festen Lackfilme wurden vom Glas zur Extraktion abgezogen.

### Extraktion:

In eine UV-undurchlässige braune Glasflasche wurden 25 g Methylenchlorid gegeben. Dazu kam eine eingewogene Menge zerkleinerten Lackfilms (4 g) und Hexadecan als interner Standard (0,2 g). Die Mischung wurde 48 Stunden gerührt und anschließend durch Gaschromatographie auf restliche Photoinitiatoren analysiert.

Die Reste der erfindungsgemäßen Photoinitiatoren konnten über der Nachweisgrenze von 0,1 % bezüglich der im Lack eingesetzten Menge nicht ermittelt werden. Im Vergleichsbeispiel mit Benzophenon als Initiator wurden dagegen 80 % wiedergefunden.
Gleiche Werte wurden nach einwöchiger Dunkellagerung der bestrahlten Lackfilme erhalten.

## Patentansprüche

1. Verfähren zur Herstellung von strahlungshärtbaren Acrylatzusammensetzungen, dadurch gekennzeichnet, daß Verbindungen mit mindestens 2 Acrylgruppen nicht radikalisch copolymerisierbare Benzophenonderivate zugesetzt werden, die mindestens eine primäre oder sekundäre Aminogruppe enthalten.

2. Verfahren gemäß Anspruch 1, dadurch gekennzeichnet, daß es sich bei den Benzophenonderivaten um solche der Formel 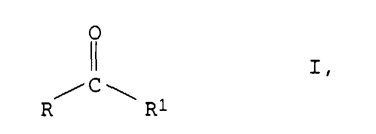 in der R für einen Phenylrest oder für R¹ steht und R¹ für den Rest 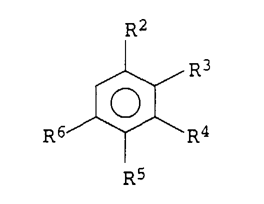 steht, wobei die Reste R² bis R⁶ unabhängig voneinander für H, eine C₁-C₄-Alkyl- oder C₁-C₄-Alkoxy-gruppe stehen und mindestens eine, maximal drei der Reste R³ bis R⁶ für eine Gruppe X steht und X eine organische Gruppe mit 1 bis 30 C-Atomen und mit einer primären oder sekundären Aminogruppe bedeutet.

3. Verfahren gemäß Anspruch 2, dadurch gekennzeichnet, daß R², R³, R⁵ und R⁶ für ein H-Atom stehen und R⁴ für X steht.

4. Verfahren gemäß Anspruch 2 oder 3, dadurch gekennzeichnet, daß X für eine aliphatische Gruppe mit 1 bis 20 C-Atomen und einer primären oder sekundären Aminogruppe steht.

5. Strahlungshärtbare Acrylatzusammensetzungen, erhältlich durch ein Verfahren gemäß einem der Ansprüche 1 bis 4.

6. Strahlungshärtbare Massen, enthaltend strahlungshärtbare Acrylatzusammensetzungen gemäß Anspruch 5.

7. Verwendung von strahlungshärtbaren Acrylatzusammensetzungen gemäß Anspruch 5 bzw. strahlungshärtbare Massen gemäß Anspruch 6 als Beschichtungsmittel.

## Claims

1. A process for preparing radiation-curable acrylate compositions, which comprises adding benzophenone derivatives which are not free-radically copolymerizable and which contain at least one primary or secondary amino group to compounds having at least 2 acrylic groups.

2. A process as claimed in claim 1, wherein the benzophenone derivatives are of the formula 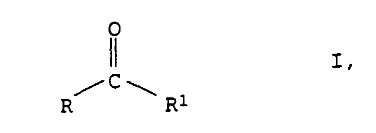 in which R is a phenyl radical or R¹ and R¹ is the radical 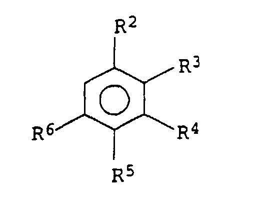 where the radicals R² to R⁶ independently of one another are H or a C₁-C₄ alkyl or C₁-C₄ alkoxy group and at least one but not more than three of the radicals R³ to R⁶ are a group X and X is an organic group having 1 to 30 carbon atoms and having one primary or secondary amino group.

3. A process as claimed in claim 2, wherein R², R³, R⁵ and R⁶ are a hydrogen atom and R⁴ is X.

4. A process as claimed in claim 2 or 3, wherein X is an aliphatic group having 1 to 20 carbon atoms and one primary or secondary amino group.

5. A radiation-curable acrylate composition obtainable by a process as claimed in one of claims 1 to 4.

6. A radiation-curable formulation comprising one or more radiation-curable acrylate compositions as claimed in claim 5.

7. The use of a radiation-curable acrylate composition as claimed in claim 5 or radiation-curable formulation as claimed in claim 6 as a coating material.

## Revendications

1. Procédé de préparation de compositions d'acrylates durcissables par rayonnement, caractérisé en ce que l'on ajoute à des composés ayant au moins deux groupements acryle des dérivés benzophénone non copolymérisables de façon radicalaire, contenant au moins un groupement amine primaire ou secondaire.

2. Procédé selon la revendication 1, caractérisé en ce que les dérivés benzophénone sont ceux de formule 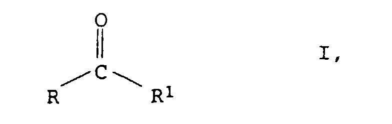 dans laquelle R représente un reste phényle ou R¹ et R¹ est mis pour le reste 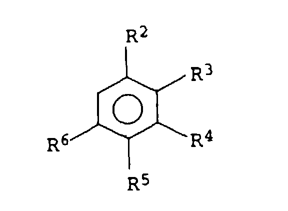 où les restes R² à R⁶ sont mis indépendamment les uns des autres pour H, un groupement alkyle en C₁-C₄ ou alcoxy en C₁-C₄ et au moins un et au plus trois des restes R³ à R⁶ sont mis pour un groupement X et X représente un groupement organique ayant 1 à 30 atomes de carbone et un groupement amine primaire ou secondaire.

3. Procédé selon la revendication 2, caractérisé en ce que R², R³, R⁵ et R⁶ sont mis pour un atome H et R⁴ est mis pour X.

4. Procédé selon la revendication 2 ou 3, caractérisé en ce que X est mis pour un groupement aliphatique ayant 1 à 20 atomes de carbone et un groupement amine primaire ou secondaire.

5. Compositions d'acrylates durcissables par rayonnement, pouvant être obtenues par un procédé selon l'une quelconque des revendications 1 à 4.

6. Masses durcissables par rayonnement, contenant des compositions d'acrylates durcissables par rayonnement selon la revendication 5.

7. Utilisation de compositions d'acrylates durcissables par rayonnement selon la revendication 5 ou de masses durcissables par rayonnement selon la revendication 6 en tant qu'agent d'enduction.
